# EUROPEAN PATENT APPLICATION

(11) **EP 4 510 145 A1**
(43) Date of publication of application: **19.02.2025**
(21) Application number: 23209090.2
(22) Date of filing: 10.11.2023
(51) Int. Cl.: G16H 40/20, G06Q 10/087

(54) **METHOD AND SYSTEM FOR PREPARING, TRACKING AND MANAGING MEDICAL DEVICES AND SUPPLIES DURING MEDICAL AND CLINICAL SETTINGS**

(30) Priority: 18.08.2023 US 202318235439
(71) Applicant: MedGEO Inc., Miami, FL 33131 (US)
(72) Inventor: VISWANATH, Arun, Barrington, 60010 (US); NAJJAR, Amel, Miami, 33131 (US); GRANT, Dane, Swayzee, 46986 (US); JOHNSON, Simon, Miami, 33131 (US)
(74) Representative: Puschmann Borchert Kaiser Klettner Patentanwälte Partnerschaft mbB

(57) **Abstract**

A method and system for preparing and managing medical devices and supplies has the steps of providing an identification database, providing an operational database, selecting, b a collection of the medical devices and supplies to be used for a medical procedure in the healthcare facility with associated identification and operational information, wherein the medical devices and supplies in the collection are selected based on a computing logic that matches the medical procedure with product information stored in the identification and operational databases, obtaining recognition features, obtaining and displaying product information, and verifying the medical devices and supplies selected for the medical procedure; and tracking and updating the use and disposal of the medical devices and supplies during and after the medical procedure.

## Description

### TECHNICAL FIELD

The present invention relates to methods and systems to optimize the workflow of medical procedures, particularly, to identify and track the use and disposition of medical assets such as surgical supplies and medical devices used in medical procedures in healthcare facilities.

### BACKGROUND OF INVENTION

Medical procedures performed in healthcare facilities often require participation of multiple medical practitioners and use of numerous medical devices and supplies with various degrees of complexity, ranging from sophisticated implantable medical devices to surgical screws, sponges, forceps, and robes. How to prepare, track, and manage the workflow of the medical devices and supplies in accordance with the requirements of the medical procedure has always been a demanding task, as both accuracy and efficiency are required. Generally, the preparation for the medical procedure must be finished within a short period of time, and all the medical devices and supplies must be readily assembled and verified for the procedure. Sometimes, a patient may have conditions that require special attention and modification to the standard procedure and devices. When a complex medical device such as an implantable device is used, a medical representative from the vendor or supplier is often present during the medical procedure to ensure the proper use of the device.

Tracking and managing the use and disposition of the medical devices and supplies during the medical procedure is equally important. Ideally, each of the medical devices and supplies used in the procedure are tracked and accounted for to ensure safety and avoid waste. After the procedure is concluded, it is important to track and manage the use and disposal of the medical devices and supplies to avoid wastes in the physical and economical senses as well as for inventory control and accurate billing for the healthcare facilities.

In the current Digital and Information Age, healthcare facilities and providers are often reluctant to embrace the newer technology and automate the workflow for the medical procedure due to concern for patient safety and a general desire to maintain traditional practice. Some operation rooms and procedures still rely on pencil and paper to record the use and disposition of the medical devices and supplies; and due to lack of prompt information sharing, there is a vast amount of information discrepancy among different departments within the healthcare facility and between the internal departments and outside healthcare facility.

For example, in the field of implantable medical devices, the current system for logging, tracking, billing, and inventory management of the implantable medical devices is not automated, optimized, and well integrated into Enterprise or Departmental Electronic Medical Record Systems (EMRs). The opportunity loss, by not having a seamless integrated real time data system for the implantable medical devices, equates to multi-million dollar loss per year per hospital in direct and indirect metric analysis, contract optimization, inventory management, electronic chart documentation, and a time lag in accounts receivable collection.

### SUMMARY OF THE INVENTION

To solve the problems in the current system, the present invention provides a method and system that can be used in various clinical settings to automatically identify parts and consumable medical devices and supplies during clinical workflows and track and manage their disposition in required systems using various optimization methods. The present invention may be used in, for examples, parts and consumables such as implantable, surgical consumables, medical consumables, and medical devices. Examples of clinical workflows can include inpatient, outpatient, acute care procedures, and can be in multiple departments like Pediatric, Orthopedic, Neurology, and spanning over multiple care pathways. The present invention provides an improved method and system for tracking various medical assets, such as implantable components and devices at the point of medical activity, for example, in the operation room (OR).

The method for preparing and managing medical devices and supplies of the present invention comprises the following steps:
(1) providing an identification database, wherein the identification database is an integrated database of two or more datasets selected from the group consisting of a first dataset of standard information on medical devices and supplies from governing agencies, a second dataset of product information from vendors and suppliers, and a third dataset of user-input information on the medical devices and supplies;
(2) providing an operational database, wherein the operational database comprises product and inventory information on medical devices and supplies associated with a healthcare facility;
(3) selecting, by one or more computing devices without user input, a collection of the medical devices and supplies to be used for a medical procedure in the healthcare facility with associated identification and operational information, wherein the medical devices and supplies in the collection are selected based on a computing logic that matches the medical procedure with product information stored in the identification and operational databases;
(4) obtaining one or more recognition features of the medical devices and supplies in the collection, obtaining and displaying product information of the medical devices and supplies in the collection based on the identification and operational databases by one or more computing devices without user input, and verifying the medical devices and supplies selected for the medical procedure; and
(5) and tracking, by one or more computing devices without user input, use and disposal of the medical devices and supplies during and after the medical procedure, and updating electronic records and the identification and operational databases based on the use and disposal of the medical devices and supplies.

In the present invention, the method for preparing and managing medical devices and supplies may further comprise updating the identification database based on periodically replicated data from the governing agencies, information from the vendors and suppliers, input from the users, or a combination thereof.

In the present invention, the method for preparing and managing medical devices and supplies may further comprise obtaining one or more recognition features on a medical device or supply and identifying the medical device or supply; retrieving, by one or more computing devices without user input, a list of medical procedures associated with the identified medical device or supply; and selecting a medical procedure to be performed from the list of the medical procedures, followed by the step of selecting the collection of the medical devices and supplies to be used for the medical procedure.

In the present invention, the method for preparing and managing medical devices and supplies may further comprise arranging, by one or more computing devices without user input, the collection of the medical devices and supplies selected for the medical procedure into one or more groups based on their related use, and tracking, by one or more computing devices without user input, use and disposal of the one or more groups of the medical devices and supplies during and after the medical procedure, and updating the electronic records and the identification and operational databases based on the use and disposal of the one or more groups of the medical devices and supplies.

In the present invention, the method for preparing and managing medical devices and supplies may further comprise providing digital access to a plurality of users for verifying the medical devices and supplies selected for the medical procedure; integrating and updating the electronic records and the identification and operational databases based on the input from the plurality of users.

In the present invention, the method for preparing and managing medical devices and supplies may further comprise alerting a user on the use and disposal of the medical devices and supplies and causing the user to replenish the medical devices and supplies or reconcile the operational database.

In the method for preparing and managing medical devices and supplies of the present invention, the recognition features may comprise bar codes, QR codes, or optical character recognition (OCR).

In the method for preparing and managing medical devices and supplies of the present invention, the medical devices and supplies selected for the medical procedure may comprise one or more implantable medical devices.

In the method for preparing and managing medical devices and supplies of the present invention, the medical procedure may comprise removal of one or more explantable medical devices from a patient, and the explantable medical devices are being tracked during and after the medical procedure and updated on the electronic records associated with the patient.

The present invention further provides a system for used in the method of preparing and managing medical devices and supplies for use in a medical procedure, comprising (1) an asset tracking subsystem, wherein the asset tracking subsystem comprises a first non-transitory medium storing instruction that, when executed, causing one or more computing devices to perform steps comprising selecting a collection of the medical devices and supplies to be used for a medical procedure in a healthcare facility, and updating related databases based on use and disposal of the medical devices and supplies during and after the medical procedure; a computing algorithm that matches the medical procedure with product information stored in related databases for selecting the collection of the medical devices and supplies; a second non-transitory medium, storing instruction that, when executed, causing one or more computing devices to perform steps comprising updating electronic records and the related databases based on the use and disposal of the medical devices and supplies, and a tangible, non-transitory storage medium storing one or more datasets related to product and inventory information on medical devices and supplies associated with the healthcare facility;
(2) a scanning subsystem, wherein the scanning subsystem comprises a third non-transitory medium storing instruction that, when executing, causing one or more computing devices to perform steps comprising obtaining one or more recognition features of the medical devices and supplies in the collection selected for the medical procedure, obtaining and displaying product information of the medical devices and supplies in the collection based on the related databases, providing an user interface to verify the medical devices and supplies selected for the medical procedure, tracking use and disposal of the medical devices and supplies during and after the medical procedure, and communicating with the first non-transitory medium to update the related databases; and
(3) an identification database subsystem, wherein the identification database subsystem comprises a tangible, non-transitory storage medium storing two or more datasets selected from the group consisting of a first dataset of standard information on medical devices and supplies from governing agencies, a second dataset of product information from vendors and suppliers, and a third dataset of user-input information on the medical devices and supplies. In the present invention, the identification database subsystem may be cloud based.

In the present invention, the system may further comprise a display screen in the scanning subsystem for displaying the product information of the medical devices and supplies in the collection based on the related databases and providing a user interface to verify the medical devices and supplies selected for the medical procedure.

In the system of the present invention, the first non-transitory medium may store instruction that, when executed, causing one or more computing devices to perform a further step of updating the identification database based on periodically replicated data from the governing agencies, information from the vendors and suppliers, input from the users, or a combination thereof.

In the system of the present invention, the third non-transitory medium in the scanning subsystem may store instruction that, when executing, causing one or more computing devices to perform further steps comprising obtaining the one or more recognition features on the medical device or supply and identifying the medical device or supply, retrieving, by one or more computing devices without user input, the list of medical procedures associated with the identified medical device or supply, and selecting the medical procedure to be performed from the list of the medical procedures, followed by the step of selecting the collection of the medical devices and supplies to be used for the medical procedure.

In the system of the present invention, the third non-transitory medium of the scanning subsystem may store instructions that, when executed, causing one or more computing devices to perform further steps of arranging, by one or more computing devices without user input, the collection of the medical devices and supplies selected for the medical procedure into one or more groups based on their related use, and tracking, by one or more computing devices without user input, use and disposal of the one or more groups of the medical devices and supplies during and after the medical procedure, and updating the electronic records and the identification and operational databases based on the use and disposal of the one or more groups of the medical devices and supplies.

In the system of the present invention, the scanning subsystem may provide digital access to a plurality of users for verifying the medical devices and supplies selected for the medical procedure, and the asset tracking subsystem may integrate and update the electronic records and the identification and operational databases based on the input from the plurality of users.

In the present invention, the recognition features may comprise bar codes, QR codes, or optical character recognition (OCR).

In the present invention, the medical devices and supplies selected for the medical procedure may comprise one or more implantable medical devices.

In the present invention, the medical procedure may comprise removal of one or more explantable medical devices from a patient, and the third non-transitory medium in the scanning subsystem may store instructions that, when executed, causing one or more computing devices to perform steps further comprising tracking the explantable medical devices during and after the medical procedure and updating the electronic records associated with the patient.

The present invention has a positive impact on multiple stakeholders. Hospitals benefit from accurate asset utilization and timely ordering of required surgical parts, as the availability of surgical assets like instruments is the second biggest cause of delays in an operating room. The present invention also benefits patients. By enabling timely and accurate information about implants in the EMR, the system enables easier tracking in case of recalled medical devices and transfer of information between hospitals. In addition, the present invention enables the hospital to be systematically compliant with the UDI Compliance Policies as outlined in the UDI Compliance Policies and UDI Rule Compliance by the FDA.

### BRIEF DESCRIPTION OF THE DRAWINGS

Figure 1 shows the structure and workflow of the method and system for preparing and managing medical devices and supplies in the present invention.
Figure 2 is an enlarged view showing the data structure of the identification database subsystem 300 of Figure 1 in the present invention.
Figure 3A shows the workflow in Example 1 using the method and system for preparing and managing medical devices and supplies of the present invention; and Figure 3B shows the workflow in a block diagram.
Figure 4A shows the workflow in Example 2 using the method and system for preparing and managing medical devices and supplies of the present invention, and Figure 4B shows the workflow in a block diagram.

Reference numbers in the figures refer to the following structure and components of the present invention: 100- asset tracking subsystem, 101, 102, 103, 104, 105, 106, 107, 108 - interfaces; 110-application services; 120- algorithms/logics; 130- integration services; 140- operational database; 200-scanning subsystem; 201- computer; 202- handheld device/mobile phone; 203- tablet; 300- identification database subsystem; , 301, 302, 303, 304, 305, 306, 307, 308, 309, 311, 312, 401, 402, 403, 404, 405, 406, 407, 408, 409, 411, 412 - various steps in the Examples 1 and 2; 330- third dataset, curated/user augmented dataset; 320- second dataset, supplier parts identification database; 310- first dataset, data from government agencies; 410- enterprise resource planning (ERPs); 420- electronic medical records (EMRs) or electronic health records (EHRs).

### DETAILED DESCRIPTION OF THE INVENTION AND EMBODIMENTS

The present invention is further explained in conjunction with the drawings and examples, but the scope of protection of the present invention shall not be limited thereby.

In the present invention, a "medical procedure" or "medical procedures" refer to any practice of a health practitioner that involves a combination of special skills or abilities and may require one or more medical devices or supplies. A medical procedure may be surgical or non-surgical, preventive, rehabilitating, diagnostic, therapeutic, anesthesia, or propaedeutic.

In the present invention, a "healthcare facility" or "healthcare facilities" refer to places and entities that provide medical care, including but not limited to, hospitals, medical offices, medical centers, urgent care centers, and nursing centers or homes.

In the present invention, a "medical device" or "medical devices" refer to the same term as defined under Section 201(h) of the Food, Drug & Cosmetic Act in the United States and regulated by the FDA. The medical device can range from simple tongue depressors and bedpans to complex programmable pacemakers, and closed loop artificial pancreas systems. Additionally, medical devices include in vitro diagnostic (IVD) products, such as reagents, test kits, and blood glucose meters. Certain radiation-emitting electronic products that have a medical use or make medical claims are also considered medical devices. Examples of these include diagnostic ultrasound products, x-ray machines and medical lasers. Medical devices includes consumable, implantable, and explantable medical devices in the present invention.

In the present invention, "medical supplies" or "surgical supplies" refer to any other tool or material that are not regulated as medical devices by the FDA but nevertheless used during or in connection with a medical procedure.

In the present invention, "medical assets" or "assets" refer to products that include medical equipment, implants, consumables, and other medical devices that are used during the medical procedure. Medical assets include both medical devices and supplies in the present invention.

In the present invention, "electronic records (ER)" include electronic medical records (EMR), electronic health records (EHR), and enterprise resource planning records (ERPs), that are commonly used in the departments in a healthcare facility.

In the present invention, a "surgery schedule" refers to a list of all surgeries scheduled for a period, such as a day, week, or month, organized by the operating rooms where they are performed.

In the present invention, a "case" refers to a single instance of a medical procedure scheduled for a particular patient.

In the present invention, a "medical or healthcare practitioner" or "medical or healthcare practitioners" refers to those participants in the medical procedure with a combination of special skills or abilities. Examples of medical or healthcare practitioners include physicians, surgeons, nurses, medical consultants, and surgery assistants.

In the present invention, a "clinical user" is a user of the present invention that is usually associated with a healthcare facility that uses the present invention to identify and track medical assets. In contrast, a "rep user" is a user of the present invention that is a representative from suppliers of the medical assets.

The medical record number (MRN) is usually used to identify a patient or a particular visit of a patient within a healthcare facility.

In the present invention, an "interface" refers to a connection between software applications, application and database, or application and user.

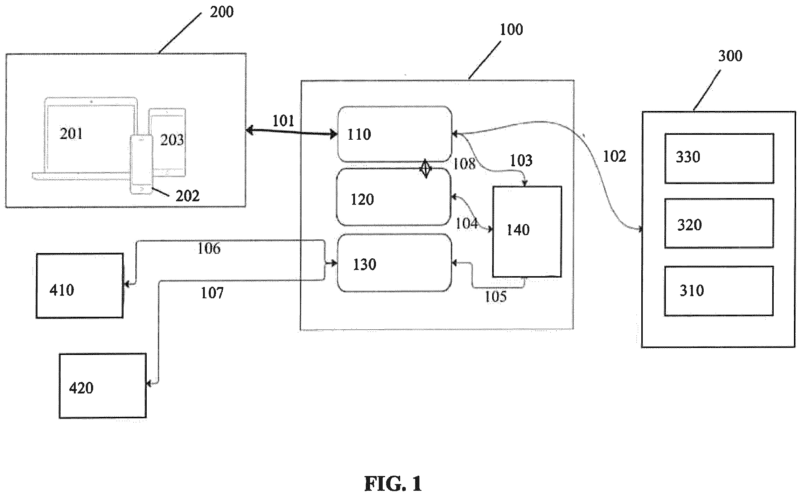

As shown in Figure 1, the system of the present invention is a computer-implemented system that comprises the following three subsystems: an asset tracking subsystem 100, a scanning subsystem 200, and an identification database subsystem 300.

The asset tracking subsystem 100 is a cloud-deployed or on-premised subsystem of software services with the ability to integrate with multiple data sources and utilizes multitude of algorithms to optimize part identification and disposition. Subsystem 100 further comprises application services 110, algorithms 120, integration services 130, and operational database 140. The subsystem 100 also provides mechanism to integrate with multitude of upstream and downstream systems like Electronic Medical Record systems (EMRs) 420 and Enterprise Resource Planning Systems (ERPs) 410, shown as integration services 130, through interfaces 106 and 107, respectively.

Application Services 110 are a set of software services that interface with scanning applications to identify an asset.

Algorithms or logic 120 are software logic that provides the intelligence to utilize the most appropriate data source to identify a given asset. The component also provides intelligence to dispose of the asset based on the asset type, for example, based on the asset type, the algorithm automatically decides if the asset was implanted in the patient or discarded.

Integration Services 130 are a set of software services that implement the protocols to integrate with EMRs 420 and ERPs 410.

Operational database 140 stores transactional information specific to a hospital that track the workflow of a case.

Scanning subsystem 200 is an application that can run on devices of various form factors including but not limited to handheld phones 202, tablets 203, computers 201, and table mounted devices like scanner stands and holders.

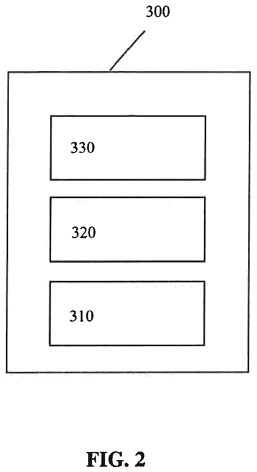

As shown in Figs. 1 and 2, the identification database subsystem 300 is a federated data source that combines information from multitude of data sources, comprising a first dataset 310 containing data available from governing bodies such as the GUDID from FDA and similar guidelines from EU governing bodies; a second dataset 320 containing data from vendors and supplier that may have supplier provided device identifiers and is structured and organized from multiple vendors; and a third dataset 330 containing data from clinically curated data sets from subject matter exports (SMEs), which may use user curated device identifiers created by hospital users on the fly. The identification database subsystem 300 is a database of curated and harmonized device identifiers from multiple sources. The database may be cloud deployed with inbuild support for redundancy and multi-tenancy, and it is reliable, highly available, and contains expandable data structure to accommodate custom attributes.

As shown in Figure 1, interface 101 shows the connection between scanning subsystem 200 and application service 220. Interface 101 is over a web protocol, such as https, and sends information, including asset number from a barcode or QR code, and retrieves the product information. Interface 102 shows the connection between application service 110 and identification database subsystem 300 to identify the medical asset. Interface 103 shows the connection between which add assets to a particular case. Also stores and retrieves states of a case (like open, approved, closed).

Interface 104 shows that algorithms 120 use data in the operational database 140 to decide what type of procedure is being done and recommends to the application service 110 via interface 108 the right data source to use and the type of disposition.

Interface 105 shows that integration Services 130 connects to the operational database 140 to store surgery schedule and retrieve case data, such as patient or asset information, so that it can be sent to ERPs and EMRs

Interface 106 shows that integration services 130 connects to ERPs 410 such as supply chain applications to retrieve information on available supplies and update asset utilization based on what's used in the case.

Interface 107 shows that integration services 130 connects to EMR 420 to receive surgery schedule and to send case details like assets used. The system of the present invention may automatically identify a part, utilizing standard identification mechanisms like barcode, QR code, printed or etched characters by comparing them with databases provided by governing bodies (like FDA, European Medical Agencies) and by utilizing curated datasets.

As shown in Figure 1, the system of the present invention may collaborate between multiple users to create, review, and reconcile medical assets to ensure precise representation of physical assets are present in disparate systems including Electron Medical Records (EMRs), Electronic Health Records (EHRs), billing, and Asset Tracking & Management systems (ERPs). The collaboration mechanism can be real-time and in the synchronous mode with two or more participating users, or in an asynchronous mode using prioritized worklists.

In the present invention, the identification database 300 is aggregation of multivariate data sources which include periodic replication of publicly available datasets from governing bodies (like FDA, European Medical Agencies) (310), supplier provided datasets, distributor provided datasets, retailer provided datasets (320), and user augmented datasets (330). Further, the database can be optimized through the process of clinical review, correction, and curation.

The system of the present invention may assign parts manually or automatically into groups and subgroups to enable optimized management and reconciliation. The grouping may include identification of products used to arrange, assemble, or segregate a set of parts, for examples, surgical trays, medical or utility carts, and mobile or stationary supply cabinets.

In the present invention, products used for the grouping can themselves be automatically identified, organized, and reconciled using the process as described.

The system of the present invention may track disposition and utilization of parts manually or automatically or set of parts, for examples, single surgical drapes, box of surgical drapes, case of multiple boxes of surgical drapes. Further, the system provides a mechanism to digitally track usage and wastage of various parts and consumables.

The system of the present invention may identify, track, and manage explanted parts and implants. Identification of the explanted part can be by looking up various data sources like Electronic Medical Records, Electronic Health Records and tracking or record keeping could be done through storing an image of the explanted part and linking them to various patient records.

The system of the present invention may use workflow schedule information (for examples, surgical schedule and procedure schedule) ingested from an external system to associate medical devices/parts/implants with a patient identified (For examples, by MRN or Patient ID).

The system of the present invention allows seamless real-time implantable data upload into the healthcare system in the operating room and immediate real time billing and logging inventory management allowing hospital systems to maximize personal efficiency, maximize collections on billable healthcare implantable, as well as date mining their own institutional system on implantable to look for opportunities to cut waste and optimize collections.

The system also creates an intelligent database that can then be utilized to help institutions leverage insurance contracts to ensure market competitiveness. The system described herein utilizes the various identification mechanisms like barcode, QR codes and Optical Character Recognition (OCR) and seamlessly integrates, in a HI PAA protected encryption manner, to the hospital electronic health chart system. This allows for real time data log data entry, real time billing inventory management, maximizing critical resource personal to ensure patient and hospital system optimization, and optimized data collection in the health care system the equates to millions of dollars of savings a year per hospital.

The system of the present invention also allows vendor inventory management with the ability to track, in real time, product utilization and facilitate strategic resource allocation.

The present invention is further explained in the following examples and figures. However, the scope of protection for the present invention shall not be limited to the examples, as one of ordinary skills in the art may change or modify the examples without departing from the scope of the present invention.

### Example 1:

Example 1 shows the workflow using the system of the present invention to identify and track implantable medical devices in a medical procedure performed at an operation room.

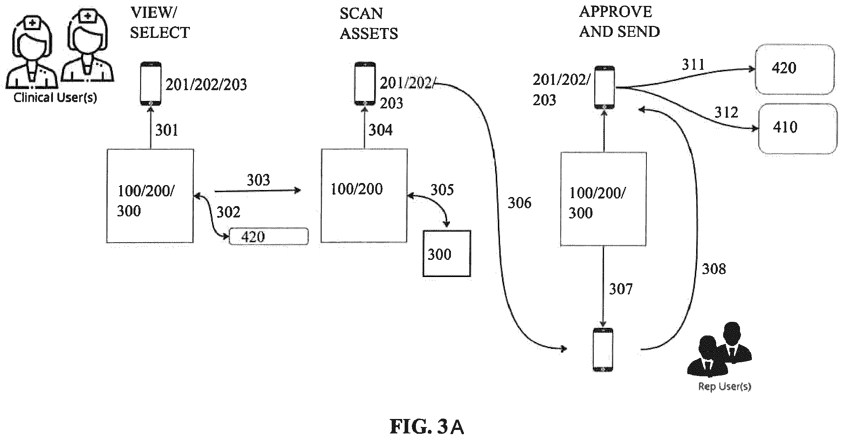

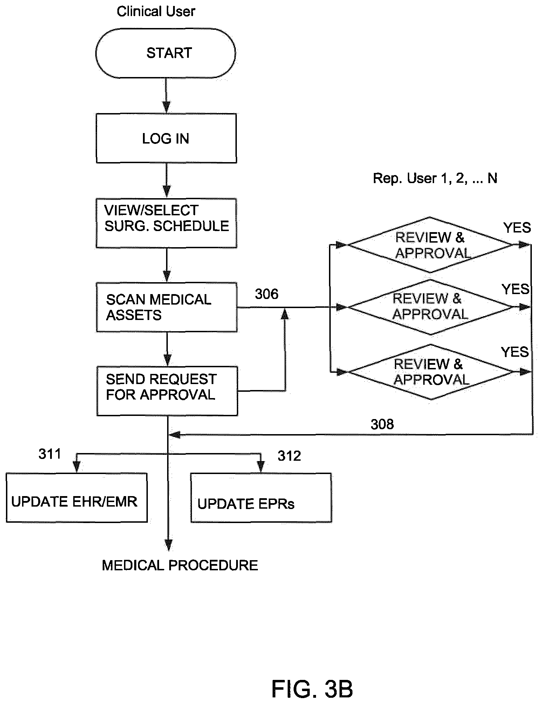

As shown in Figures 3A and 3B, the workflow starts in the first step where clinical user logs in to the system of the present invention using a desktop 201, a mobile device 202, or a tablet 203, and views the surgery schedule for the respective operation room for the day. In the second step, clinical user scans the set of medical assets to be used in the surgery. In the third step, in the workflow post scanning, all the relevant medical assets are getting approval from the supplier representative, and information on the assets are sent as a record to external systems such as EMR or ERP.

In step 301, the user selects the right case from the surgical schedule. In step 302, the system of the present invention retrieves the surgery schedule from the EMR/EHR and displays the list to the user. Once the user selects a "Case" to work on, the app automatically moves to scanning mode as shown in step 303. In step 304, the user starts scanning assets using the scanning application 200. Through step 305, system application 110 uses intelligent mechanisms like type of procedure and type of assets to find the right data source within the identification database 300 to identify the asset.

Through step 306, and once the clinical user has scanned all assets that had been utilized during the case, they send a notification to supplier representatives to verify the list of assets used during the case. It can be to more than 1 representative as a case could involve assets from multiple suppliers. Through step 307, each Rep User individually pulls the list of assets they had supplied for this case and approves the list using the system of the present invention. Through step 308 and once all involved Rep Users approve the asset used in the case, a notification is sent to the clinical user about the approval being complete.

Through step 309, clinical user open the approved case on the scanning app and sends the case information to configured destinations, including in step 311, clinical user sends case and asset information to EMR/EHR 420, and in step 312, clinical user sends asset utilization information to ERP 410.

### Example 2:

Example 2 shows the collaboration workflow between the clinical users (nurses at the hospital) and rep user from the vendor of the medial device.

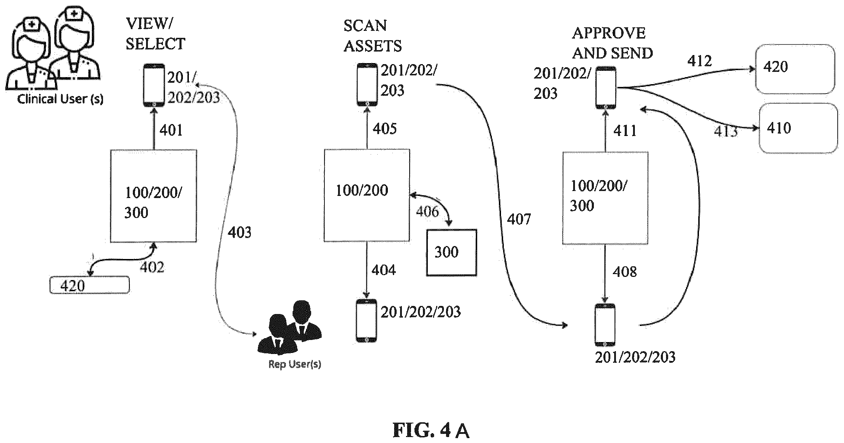

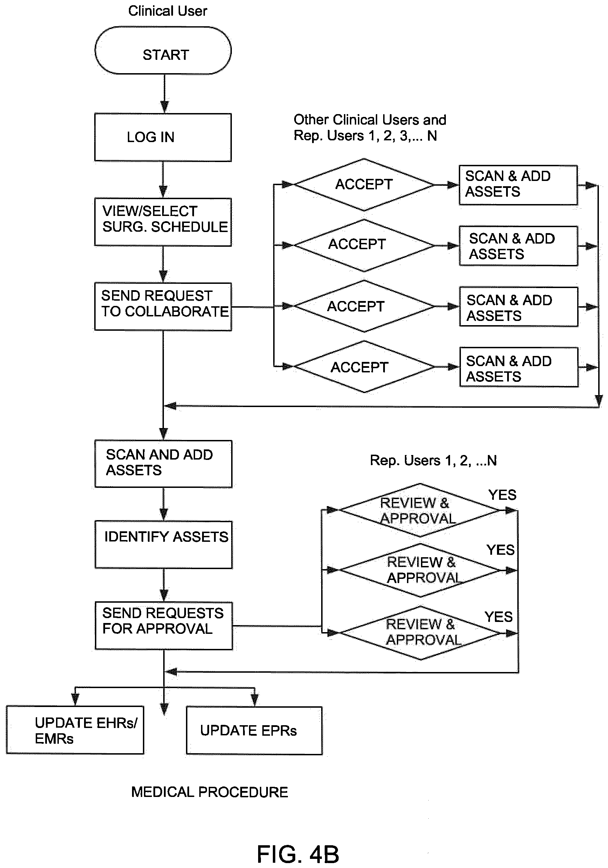

As shown in Figures 4A and 4B, clinical user logs in to the system of the present invention using a desktop 201, a mobile device 202, or a tablet 203, and views the surgery schedule for respective operation room for the day. In the step in the workflow, user scans the set of assets used in the surgery. Then, in the workflow post scanning, all relevant assets are sent to and get approval from the supplier representative and the information on the assets are sent as a record to external systems like EMR 420 or ERP 410 for updating.

In step 401, the user selects the right case from the schedule. In step 402, the system of the present invention retrieves the surgery schedule from the EMR/EHR 420 and displays the list to the user. In step 403, clinical user invites other clinical user(s) and/or rep user(s) to collaborate and add assets used in the case through the system of the present invention. In step 404, rep users accept the invite and start scanning assets from respective vendors simultaneously. In step 405, the clinical user also has the ability to add assets through the scanning process. In step 406, the system of the present invention 100/200 uses intelligent mechanisms like type of procedure and type of assets to find the right data source within the identification database 300 to identify the asset.

In step 407, once the clinical user has scanned all assets that were utilized during the case, they send a notification to supplier representatives to verify the list of assets used during the case. This can be to more than one representative as a case could involve assets from multiple suppliers. In step 408, each rep user individually pulls the list of assets they had supplied for this case and approves the list using the system of the present invention. In step 409, once all involved rep users approve the asset used in the case, a notification is sent to the clinical user about the approval being complete. In step 411, clinical user opens the approved case on the scanning application 200 and sends the case information to configured destinations, followed by step 412 where clinical user sends case and asset information to EMR/EHR 420 and step 413 where clinical user sends asset utilization information to ERP 410.

## Claims

1. A method for preparing and managing medical devices and supplies for use in a medical procedure, comprising
providing an identification database, wherein the identification database is an integrated database of two or more datasets selected from the group consisting of a first dataset of standard information on medical devices and supplies from governing agencies, a second dataset of product information from vendors and suppliers, and a third dataset of user-input information on the medical devices and supplies,
providing an operational database, wherein the operational database comprises product and inventory information on medical devices and supplies associated with a healthcare facility,
selecting, by one or more computing devices without user input, a collection of the medical devices and supplies to be used for a medical procedure in the healthcare facility with associated identification and operational information, wherein the medical devices and supplies in the collection are selected based on a computing logic that matches the medical procedure with product information stored in the identification and operational databases,
obtaining one or more recognition features of the medical devices and supplies in the collection, obtaining and displaying product information of the medical devices and supplies in the collection based on the identification and operational databases by one or more computing devices without user input, and verifying the medical devices and supplies selected for the medical procedure,
tracking, by one or more computing devices without user input, use and disposal of the medical devices and supplies during and after the medical procedure, and updating electronic records and the identification and operational databases based on the use and disposal of the medical devices and supplies.

2. The method of claim 1, further comprising
updating the identification database based on periodically replicated data from the governing agencies, information from the vendors and suppliers, input from the users, or a combination thereof.

3. The method of claim 1, further comprising
obtaining one or more recognition features on a medical device or supply and identifying the medical device or supply,
retrieving, by one or more computing devices without user input, a list of medical procedures associated with the identified medical device or supply,
selecting a medical procedure to be performed from the list of the medical procedures, followed by the step of selecting the collection of the medical devices and supplies to be used for the medical procedure.

4. The method of claim 1, further comprising
arranging, by one or more computing devices without user input, the collection of the medical devices and supplies selected for the medical procedure into one or more groups based on their related use, and
tracking, by one or more computing devices without user input, use and disposal of the one or more groups of the medical devices and supplies during and after the medical procedure, and updating the electronic records and the identification and operational databases based on the use and disposal of the one or more groups of the medical devices and supplies.

5. The method of claim 1, further comprising
providing digital access to a plurality of users for verifying the medical devices and supplies selected for the medical procedure, and
integrating and updating the electronic records and the identification and operational databases based on the input from the plurality of users.

6. The method of claim 1, further comprising
alerting a user on the use and disposal of the medical devices and supplies, and
causing the user to replenish the medical devices and supplies or reconcile the operational database.

7. The method of claim 1, wherein the recognition features comprise bar codes, QR codes, or optical character recognition (OCR).

8. The method of claim 3, wherein the recognition features comprise bar codes, QR codes, or optical character recognition (OCR).

9. The method of claim 1, wherein the medical devices and supplies selected for the medical procedure comprise one or more implantable medical devices.

10. The method of claim 1, wherein the medical procedure comprises removal of one or more explantable medical devices from a patient, and the explantable medical devices are being tracked during and after the medical procedure and updated on the electronic records associated with the patient.

11. A system for preparing and managing medical devices and supplies for use in a medical procedure, comprising
(1) an asset tracking subsystem, wherein the asset tracking subsystem comprises
a first non-transitory medium storing instruction that, when executed, causing one or more computing devices to perform steps comprising
selecting a collection of the medical devices and supplies to be used for a medical procedure in a healthcare facility, and
updating related databases based on use and disposal of the medical devices and supplies during and after the medical procedure,
a computing algorithm that matches the medical procedure with product information stored in related databases for selecting the collection of the medical devices and supplies,
a second non-transitory medium, storing instruction that, when executed, causing one or more computing devices to perform steps comprising
updating electronic records and the related databases based on the use and disposal of the medical devices and supplies, and
a tangible, non-transitory storage medium storing one or more datasets related to product and inventory information on medical devices and supplies associated with the healthcare facility;
(2) a scanning subsystem, wherein the scanning subsystem comprises
a third non-transitory medium storing instruction that, when executing, causing one or more computing devices to perform steps comprising
obtaining one or more recognition features of the medical devices and supplies in the collection selected for the medical procedure,
obtaining and displaying product information of the medical devices and supplies in the collection based on the related databases,
providing a user interface to verify the medical devices and supplies selected for the medical procedure,
tracking use and disposal of the medical devices and supplies during and after the medical procedure, and
communicating with the first non-transitory medium to update the related databases; and
(3) an identification database subsystem, wherein the identification database subsystem comprises a tangible, non-transitory storage medium storing two or more datasets selected from the group consisting of a first dataset of standard information on medical devices and supplies from governing agencies, a second dataset of product information from vendors and suppliers, and a third dataset of user-input information on the medical devices and supplies.

12. The system of claim 11, wherein the identification database subsystem is cloud based.

13. The system of claim 11, further comprising
a display screen in the scanning subsystem for displaying the product information of the medical devices and supplies in the collection based on the related databases and providing a user interface to verify the medical devices and supplies selected for the medical procedure.

14. The system of claim 11, wherein the first non-transitory medium storing instruction that, when executed, causing one or more computing devices to perform a further step of updating the identification database based on periodically replicated data from the governing agencies, information from the vendors and suppliers, input from the users, or a combination thereof.

15. The system of claim 11, wherein the third non-transitory medium in the scanning subsystem storing instruction that, when executing, causing one or more computing devices to perform further steps comprising
obtaining the one or more recognition features on the medical device or supply and identifying the medical device or supply,
retrieving, by one or more computing devices without user input, the list of medical procedures associated with the identified medical device or supply, and
selecting the medical procedure to be performed from the list of the medical procedures, followed by the step of selecting the collection of the medical devices and supplies to be used for the medical procedure.

16. The system of claim 11, wherein the third non-transitory medium of the scanning subsystem, storing instructions that, when executed, causing one or more computing devices to perform further steps of
arranging, by one or more computing devices without user input, the collection of the medical devices and supplies selected for the medical procedure into one or more groups based on their related use, and
tracking, by one or more computing devices without user input, use and disposal of the one or more groups of the medical devices and supplies during and after the medical procedure, and updating the electronic records and the identification and operational databases based on the use and disposal of the one or more groups of the medical devices and supplies.

17. The system of claim 11, wherein the scanning subsystem provides digital access to a plurality of users for verifying the medical devices and supplies selected for the medical procedure, and the asset tracking subsystem integrates and updates the electronic records and the identification and operational databases based on the input from the plurality of users.

18. The system of claim 11, wherein the recognition features comprise bar codes, QR codes, or optical character recognition (OCR).

19. The system of claim 11, wherein the medical devices and supplies selected for the medical procedure comprise one or more implantable medical devices.

20. The system of claim 11, wherein the medical procedure comprises removal of one or more explantable medical devices from a patient, and
the third non-transitory medium in the scanning subsystem storing instructions that, when executed, causing one or more computing devices to perform steps further comprising
tracking the explantable medical devices during and after the medical procedure and updating the electronic records associated with the patient.
